# EUROPÄISCHE PATENTSCHRIFT

(11) **EP 3 675 769 B1**
(45) Veröffentlichungstag und Bekanntmachung des Hinweises auf die Patenterteilung: **26.04.2023**
(21) Anmeldenummer: 18773079.1
(22) Anmeldetag: 28.08.2018
(51) Int. Cl.: A61C 8/00, A61C 3/02, A61B 17/86, A61F 2/08, A61L 27/58, A61B 17/00

(54) **DENTALSCHRAUBE**
DENTAL SCREW
VIS DENTAIRE

(30) Priorität: 28.08.2017 DE 102017119657
(43) Veröffentlichungstag der Anmeldung: 08.07.2020
(73) Patentinhaber: Botiss Biomaterials GmbH, 15806 Zossen (DE)
(72) Erfinder: BIELENSTEIN, Oliver, 10629 Berlin (DE); TADIC, Drazen, 14129 Berlin (DE); WITTE, Frank, 10589 Berlin (DE); MATTHYS, Romano, 8810 Horgen (CH)
(74) Vertreter: Friderichs, Gunther
(86) Internationale Anmeldenummer: PCT/EP2018/073094
(87) Internationale Veröffentlichungsnummer: WO 2019/042974

(56) Entgegenhaltungen:
- WO-A2-2004/112841
- DE-A1- 3 630 863
- DE-A1-102014 008 449
- DE-C1- 3 701 765
- DE-C1- 10 065 799
- DE-T2- 69 528 501
- US-A1- 2007 218 750

## Beschreibung

### Gebiet der Erfindung

Die Erfindung betrifft eine bioresorbierbare Schraube zur Fixierung eines Implantats, welche insbesondere im Dentalbereich Verwendung findet. Insbesondere betrifft die Erfindung Schrauben zur Fixierung von bioresorbierbaren Membranen und Implantaten, mit denen Knochendefekte aufgefüllt werden. Die Erfindung betrifft des Weiteren ein Set mit einem Implantat und zumindest einer bioresorbierbaren Schraube.

### Hintergrund der Erfindung

Bioresorbierbare Implantate, wie insbesondere Kollagenfolien, werden in der Praxis in der Regel mit Nägeln oder Drähten befestigt, welche nach dem Auflösen des Implantats aufwendig explantiert werden müssen.

Auf dem Markt angebotene bioresorbierbare Schrauben, wie sie beispielsweise für Knochenplatten verwendet werden, sind zumeist wenig geeignet, um Implantate im Kieferbereich zu verankern. Insbesondere sind die meisten auf dem Markt verfügbaren Schrauben dafür ausgelegt, ein massives und rigides Bauteil zu befestigen und eignen sich daher wenig zur Fixierung von dünnen biegsamen Flächengebilden, wie beispielsweise Folien oder Membranen aus Kollagen oder für poröse Formkörper.

Eine Knochenschraube mit einem abbrechbaren Antrieb ist aus dem Dokument US 2007/0218750 A1 bekannt.

### Aufgabe der Erfindung

Vor diesem Hintergrund liegt der Erfindung die Aufgabe zugrunde, eine Schraube bereitzustellen, welche zum einen bioresorbierbar ausgebildet ist und welche zum anderen eine gut handhabbare, sichere Befestigung von Implantaten ermöglicht, welche nicht aus einem massiven, rigiden Material bestehen.

### Zusammenfassung der Erfindung

Die Aufgabe der Erfindung wird bereits durch eine Schraube zur Fixierung eines Implantats nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft eine Schraube zur Fixierung eines Implantats. Insbesondere betrifft die Erfindung eine Schraube, welche im Dentalbereich, insbesondere im Bereich der oralen Chirurgie, Verwendung findet.

Im Sinne der Erfindung ist aber durchaus auch möglich, die Schraube zur Fixation von Sehnen oder Bändern oder zur Fixation kleiner Knochenfragmente, insbesondere auch in der Hand- und Fußchirurgie, einzusetzen. Denkbar ist auch eine Verwendung bei knöchernen Sehnenabrissen, bei der Fixation der Kapsel bei Ritzarthrose oder beim Einsetzen eines Spacers. Auch an der Hüfte oder Schulter oder bei der Arthroskopie am Knie könnte die Schraube Verwendung finden.

Weitere Verwendungsbereiche sind die Befestigung von Blöcken aus Knochenersatzmaterial oder von Blöcken aus gesinterten Magnesiumpartikeln und/oder gesinterten Magnesiumfasern, welche in Defektstellen eingebracht werden können und welche das Knochenwachstum fördern sollen.

Auch bei der Knochenaugmentation ist eine Verwendung denkbar. Die Schraube besteht aus einem bioresorbierbaren Material, nämlich aus Magnesium oder aus einer Magnesiumlegierung.

Bei einer bevorzugten Ausführungsform besteht die Schraube aus einer Magnesiumlegierung mit Yttrium, Zink, Mangan und/oder Kalzium, insbesondere als Hauptlegierungsbestandteile. Yttriumhaltige Magnesiumlegierungen haben verbesserte Eigenschaften, was die Korrosion angeht, d.h. die Schrauben korrodieren, verglichen mit reinem Magnesium, langsamer.

Die Schraube weist ein Gewinde zum Eindrehen in den Knochen sowie einen Kopf mit einem Antrieb und mit einer Kappe auf. Die Kappe weist eine im Wesentlichen flache Anlagefläche für das Implantat auf. Gemäß der Erfindung ist zwischen dem Antrieb und der Kappe eine Sollbruchstelle vorhanden.

Um ein einfaches Einbringen der Schraube zu ermöglichen, wird also der Antrieb mit einem Handhabungswerkzeug, wie einem elektrisch oder pneumatisch betriebenen Schraubendreher (z.B. auch einem langsam drehenden Zahnarztbohrer) oder einem mechanischen Schraubendreher, verbunden.

Antrieb und Werkzeug sind vorzugsweise derart ausgebildet, dass der Antrieb in der Werkzeugaufnahme verriegelt wird, insbesondere klemmt oder verrastet.

Die Schraube kann so über das Handhabungswerkzeug eingedreht werden und es wird nach dem Eindrehen der Antrieb durch Abbrechen an der Sollbruchstelle entfernt, ohne dass die Gefahr des Herausfallens besteht.

Gemäß einer Ausführungsform ist die Sollbruchstelle derart ausgebildet, dass, sobald die Anlagefläche der Kappe am Implantat zur Anlage kommt und so ein weiteres Eindrehen blockiert, der Antrieb durch das Torsionsmoment beim weiteren Eindrehen abbricht.

Eine weitere Möglichkeit ist ein Abbrechen des Antriebs durch ein Verkippen des Handhabungswerkzeugs. Sofern etwa die Knochenstruktur bei einem Patenten sehr weich ist und ein Ausbrechen der Schraube droht, kann der Antrieb auch mittels eines Werkzeugs abgeknipst werden.

Nach dem Abbrechen des Antriebs sitzt die vorzugsweise pilzkopfförmige Kappe flächig auf dem Implantat auf, so dass auch fragile oder dünne Implantate relativ sicher befestigt sind.

Insbesondere können auch biegsame Flächenmaterialien, wie beispielsweise Magnesium- und/oder Kollagenfolien sicher fixiert werden.

Die Sollbruchstelle ist gemäß einer bevorzugten Ausführungsform der Erfindung als Einschnürung ausgebildet, vorzugsweise als eine Einschnürung, deren kleinster Durchmesser kleiner als der kleinste Durchmesser eines Schaftes und/oder eines Gewindekerns ist. Insbesondere hat die Einschnürung den 0,70 bis 0,99fachen Durchmesser.

Es hat sich herausgestellt, dass bereits der 0,90 bis 0,99fache Durchmesser der Einschnürung gegenüber dem kleinsten Durchmesser des Schaftes oder Gewindekerns ausreichend ist, um in jedem Fall ein Abbrechen an der Sollbruchstelle zu gewährleisten, ohne dass die Gefahr besteht, dass die Schraube an einer anderen Stelle, insbesondere im Bereich eines Schaftes oder des Gewindes, bricht.

Die Kappe ist vorzugsweise plattenförmig ausgebildet. Vorzugsweise hatte die Kappe eine maximale Höhe bis zur Sollbruchstelle von weniger als 2 mm, besonders bevorzugt von weniger als 1 mm und ganz besonders bevorzugt von weniger als 0,8 mm.

Durch den abbrechbaren Antrieb kann eine Kappe mit geringer Höhe bereitgestellt werden, da diese nicht mit Formschlusselementen zum Eingriff eines Werkzeugs versehen sein muss.

Bei einer Ausführungsform der Erfindung ist der kleinste Durchmesser eines Schaftes der Schraube maximal so groß wie der Kerndurchmesser des Gewindes. Insbesondere ist der kleinste Durchmesser des Schaftes in etwa so groß wie der Kerndurchmesser des Gewindes.

Dies hat zur Folge, dass Schaft und Gewindekern zumindest abschnittweise einen Kreiszylinder bilden. Aus diesem Kreiszylinder ragen die Zähne der Gewindezüge hervor.

Knochenschrauben finden in der Regel sicheren Halt nur in der Kortikalis des Knochens. Ist nun die Kortikalis dünn ausgebildet, wie dies insbesondere bei älteren Patienten häufig vorkommt, kann es dazu kommen, dass beim vollständigen Eindrehen der Schraube nur noch der Schaft, nicht aber das Gewinde in der Kortikalis verankert ist. Das Gewinde ist also bis in die Spongiosa durchgedreht und hat dort keinen Halt.

Dadurch, dass nunmehr aber ein Loch in der Kortikalis ist, dessen Durchmesser in etwa dem Kerndurchmesser des Gewindes entspricht und welches also lediglich mit dem Gewinde der Schraube korrespondierende Gewindezüge aufweist, ist die Schraube, auch wenn sie "durchgedreht" ist, zumindest gegen axiales Verschieben gesichert, fällt also auch ohne, dass das Gewinde in der Kortikalis verklemmt ist, nicht heraus.

Gemäß der Erfindung hat das Gewinde eine selbstschneidende Spitze.

Bei einer weiteren Ausführungsform der Erfindung ist die Schraube derart ausgebildet, dass diese eingeschlagen werden kann. Insbesondere hat eine Spitze der Schraube einen Kegelwinkel von unter 60°, bevorzugt von unter 40°.

Das Gewinde ist gemäß einer Ausführungsform einzügig ausgebildet. Denkbar ist aber auch die Ausgestaltung mit einem mehrzügigen, insbesondere zweizügigen, Gewinde, insbesondere bei einer Ausführungsform, bei welcher die Schraube auch eingeschlagen werden kann, statt eingeschraubt zu werden.

Erfindungsgemäß hat die Schraube zwar eine selbstschneidende Spitze, welche beim Eindrehen der Schraube in das Knochengewebe Gewindezüge einschneidet.

Dennoch wird vorzugsweise vor dem Eindrehen der Schraube vorgebohrt, insbesondere mit einem Bohrer, dessen Durchmesser sich maximal um 25 % vom Kerndurchmesser des Gewindes unterscheidet.

Die selbstschneidende Spitze ist aber gemäß einer Ausführungsform derart ausgebildet, dass nur in einem Randbereich, nicht aber in einem Zentrum der Spitze eine Schneide vorhanden ist.

Insbesondere erstreckt sich die zumindest eine Schneide der selbstschneidenden Spitze über einen Bereich von 10 bis 80 %, vorzugsweise von 20 bis 60 % des Außendurchmessers des Gewindes.

Es muss so nicht die gesamte Bohrtätigkeit durch das selbstschneidende Gewinde der Schraube geleistet werden, was die auftretenden Drehmomente beim Eindrehen reduziert.

Weiter kann, wie es bei einer Ausführungsform vorgesehen ist, die Schraube mit einem Zentrierkegel versehen sein, in dessen Bereich keine Schneide angeordnet ist. Die Schraube lässt sich so gut ins Bohrloch einbringen und zentrieren.

Bei einer anderen Ausführungsform erstreckt sich die selbstschneidende Spitze bis zum vorderen Ende der Schraube. Diese Ausführungsform ist besonders für eine einschlagbare Schraube geeignet.

Die Kappe hat gemäß einer Ausführungsform einen Durchmesser von 1 bis 6 mm, vorzugsweise von 2 bis 4 mm.

Die Kappe hat vorzugsweise den 1,5 bis 5fachen, besonders bevorzugt den 2 bis 4fachen Durchmesser des Schaftes und/oder des Gewindekerns.

Die Steigung des Gewindes beträgt vorzugsweise 0,5 bis 1,6 mm. Bei einer Ausführungsform, bei welcher die Schraube eingeschlagen werden kann, beträgt die Steigung vorzugsweise über 1,6 mm, insbesondere 1,6 bis 2,5 mm.

Erfindungsgemäß sind die Zähne des Gewindes außen flach oder abgerundet ausgebildet.

Die Zähne des Gewindes sind derart gestaltet, dass der Zahngrund abgerundet in die Zahnflanken übergeht.

Insbesondere ist das Gewinde als ein- oder zweigängiges Trapezgewinde mit abgerundetem Zahngrund ausgebildet.

Vorzugsweise gegen die Zahnflanken abgerundet in die Zahnspitzen über.

So werden scharfe Kanten des Gewindes vermieden, die nach der Implantation schneller korrodieren.

Insbesondere haben die Zähne mindestens die 0,5fache, vorzugsweise die 0,7fache Breite des Zahngrundes.

Bei einer Weiterbildung der Erfindung weist die Schraube eine Beschichtung auf. Insbesondere weist die Schraube eine Beschichtung aus Magnesiumfluorid auf. Eine derartige passivierende Beschichtung, die in der Initialzeit nach dem Einsetzen die Korrosion hemmt, lässt sich auf einfache Weise durch Eintauchen einer Schraube aus Magnesium oder einer Magnesiumlegierung in Flusssäure bereitstellen.

Insbesondere wird eine verstärkte Korrosion aufgrund einer höheren Chloridkonzentration einer in Kochsalzlösung gequollenen Kollagenmembran, welche mit der Schraube befestigt wird, reduziert.

Auch kann die Bildung von galvanischen Zellen aufgrund angrenzender Metalle und die damit einhergehende erhöhte initiale Korrosion verringert werden.

Gemäß der Erfindung hat die Kappe eine vorzugsweise im Wesentlichen flache Anlagefläche für das Implantat, wobei sich der Schaft zur Kappe hin, welche ggf. zusammen mit dem Antrieb den Kopf der Schraube bildet, verdickt.

Der Schaft ist also in einem Bereich direkt unterhalb der Kappe dicker als im daran angrenzenden Bereich in Richtung Gewinde ausgebildet.

Dies führt dazu, dass am Ende des Eindrehvorgangs der verdickte Bereich an der Oberseite des Implantats zur Anlage kommt, so dass das notwendige Drehmoment für ein weiteres Hineindrehen sprunghaft größer wird und die Schraube vom Benutzer nicht weiter eingedreht wird.

So ist sichergestellt, dass die Anlagefläche der Kappe nicht derart stark beim Eindrehen auf das Implantat bedrückt wird, dass dieses beschädigt wird.

Dies gilt insbesondere für Implantate, welche als biegsame Flächengebilde ausgebildet sind.

Durch die Verdickung unterhalb der Kappe wird gleichsam zwischen dem Knochen und der Anlagefläche ein Spalt geschaffen, in welchem das Flächengebilde sitzt.

Der verdickte Bereich ist insbesondere derart ausgebildet, dass der Schaft angrenzend zum Kopf einen konusförmigen Abschnitt aufweist. Hierdurch kommt es zu einem Verklemmen der Schraube am Ende des Einschraubvorgangs, wobei die Kappe der Schraube noch von der Kortikalis beabstandet ist und daher eine Ausstanzung des Implantats durch die Kappe vermieden wird.

Durch einen konusförmigen Abschnitt kommt es also im Unterschied zu einer Stufe in der Regel beim Implantat zu einer Aufweitung des Bohrlochs bis sich die Schraube festklemmt. Hierdurch werden die Drehmomente, die beim Eindrehen der Schraube über die Anlagefläche im Sinne einer Reibkupplung an das Implantat übertragen werden können und so zu einer Ausstanzung führen können, reduziert.

Der konusförmige Abschnitt kann insbesondere kegelstumpfförmig ausgebildet sein.

Bei einer Weiterbildung der Erfindung wird der Winkel des konusförmigen Abschnitts in einen Übergangsbereich der Kappe hin, bezogen auf eine Mittelachse, steiler. Insbesondere ist der Übergangsbereich zwischen konusförmigem Abschnitt und angrenzender Kappe als Radius ausgebildet.

Hierdurch steigt der Durchmesser des Schaftes direkt vor der Anlagefläche stark an, so dass es, spätestens wenn dieser Bereich mit dem Implantat in Kontakt kommt, zu einem Festsetzen der Schraube kommt.

Der Übergangsbereich hat vorzugsweise eine Länge von 0,5 bis 2 mm (entlang der Mittelachse der Schraube). So ist die Schraube besonders gut zum Festlegen von Flächengebilden als Implantate geeignet.

Der konusförmige Abschnitt kann gemäß einer Ausführungsform der Erfindung, bezogen auf eine Mittelachse, eine Mantellinie aufweisen, die in einem (gemittelten) Winkel von 20 bis 40°, vorzugsweise von 35 bis 45° zur Mittelachse der Schraube verläuft. Vorzugsweise hat der konusförmige Abschnitt eine axiale Länge von 0,2 bis 10 mm, vorzugsweise von 0,4 bis 0,6 mm.

Vorzugsweise hat der konusförmige Bereich einen größten Durchmesser, der 1,1 bis 2,5, besonders bevorzugt 1,3 bis 1,8 mal größer ist als der kleinste Durchmesser des Schaftes.

Die Erfindung betrifft des Weiteren ein Set mit zumindest einer Schraube, wie sie vorstehend beschrieben wurde und einem Implantat.

Insbesondere ist das Implantat als biegsames Flächengebilde, insbesondere als Kollagenmembran und/oder Magnesiumfolie ausgebildet.

Das Set kann des Weiteren, wie es gemäß einer Ausführungsform vorgesehen ist, einen Vorbohrer umfassen, welcher vorzugsweise in etwa den Durchmesser des Schafts und/oder des Kerndurchmessers des Gewindes der Schraube aufweist.

Je nach Zustand der Knochensubstanz kann mit einem Vorbohrer vorgebohrt werden, der einen kleineren Durchmesser als der Kerndurchmesser des Gewindes hat oder es kann auf ein Vorbohren sogar verzichtet werden, insbesondere bei der einschlagbaren Ausführungsform der Schraube.

Zu dem Set kann des Weiteren noch ein Halter für eine Mehrzahl von Schrauben und/oder eine Schablone zum Positionieren des Vorbohrers gehören. Der Halter dient der Zwischenlagerung der Schrauben und ist vorzugsweise sterilisierbar. Insbesondere besteht der Halter aus einem metallfreien sterilisierbaren Material, z.B. aus einem Polysiloxan. So können die Schrauben steril aufgenommen werden, ohne diese anzufassen.

Weiter kann das Set ein Zwischenstück für eine Schraube umfassen, des eine Kupplung zum Verbinden mit einem Handstück zum manuellen Eindrehen der Schrauben oder mit einem Dentalbohrer aufweist.

### Kurzbeschreibung der Zeichnungen

Die Erfindung soll im Folgenden bezugnehmend auf Ausführungsbeispiele anhand der Zeichnungen Fig. 1 bis Fig. 9 näher erläutert werden.
Fig. 1 ist eine Seitenansicht einer ersten Ausführungsform einer Schraube, welche insbesondere für die orale Chirurgie, und zwar insbesondere zum Befestigen von Flächengebilden, wie Kollagenmembranen oder Magnesiumfolien, vorgesehen ist.
Fig. 2 ist eine Draufsicht auf die Spitze der Schraube.
Fig. 3 ist eine Schnittansicht entlang A-A gemäß Fig. 2.
Fig. 4 ist eine Schnittansicht entlang B-B der Fig. 1.
Fig. 5 ist eine Seitenansicht eines anderen Ausführungsbeispiels einer Schraube. Diese ist länger ausgebildet und insbesondere zur Befestigung von Blöcken, mit denen Knochendefekte aufgefüllt werden, vorgesehen.
Fig. 6 ist eine vergrößerte Ansicht der Fig. 5.
Fig. 7 zeigt, schematisch dargestellt, wie ein Implantat mit einer der Schrauben befestigt werden kann.
Fig. 8 und Fig. 9 zeigen eine einschlagbare Ausführungsform einer Schraube.
Fig. 10 und Fig. 11 zeigen ein mit einem Dentalbohrer oder Handschraubendreher koppelbares Zwischenstück für eine der Schrauben.

### Detaillierte Beschreibung der Zeichnungen

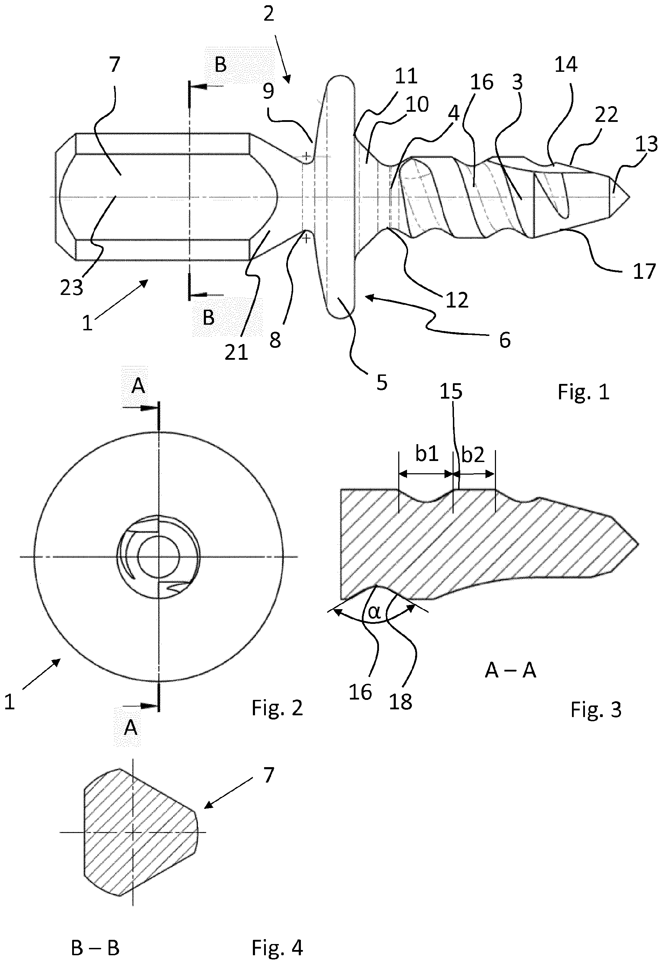

Fig. 1 ist eine Seitenansicht einer Schraube 1 gemäß eines ersten Ausführungsbeispiels der Erfindung.

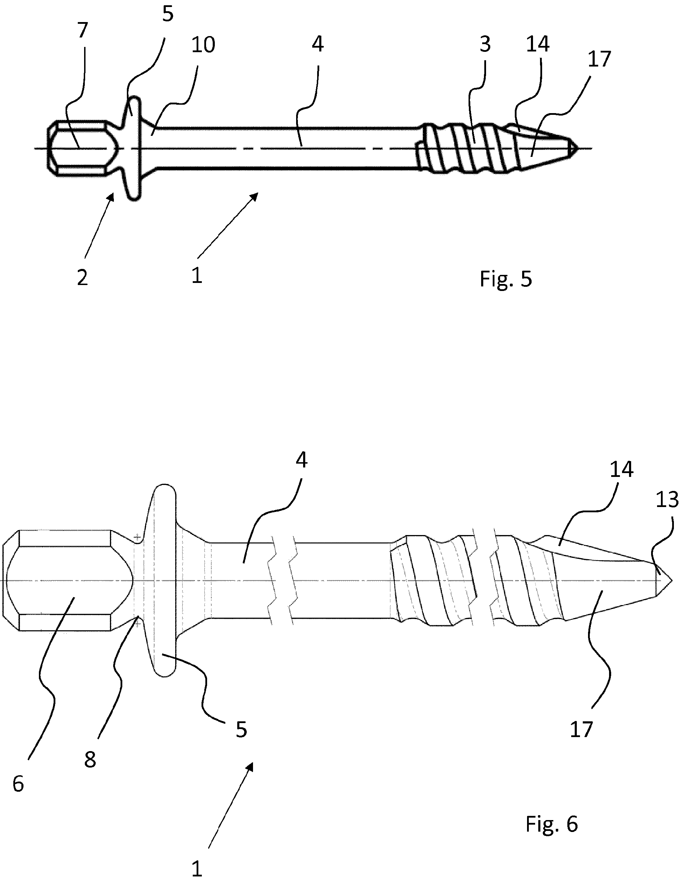

Die Schrauben haben generell vorzugsweise eine Gesamtlänge (einschließlich Kopf 2 mit Antrieb 7) von 3 bis 20 mm. Die hier gegenüber Fig. 5 und Fig. 6 dargestellte kürzere Version hat vorzugsweise eine Gesamtlänge von 5 bis 10 mm.

Die Schraube 1 umfasst einen Kopf 2, welcher aus der Kappe 5 und dem Antrieb 7 besteht. An den Kopf 2 grenzt ein Schaft 4, der in diesem Ausführungsbeispiel unmittelbar in das Gewinde 3 übergeht.

Die Kappe 5 ist plattenförmig ausgestaltet und in diesem Ausführungsbeispiel an der Oberseite 9 abgerundet.

Die Unterseite bildet eine vorzugsweise ebene Anlagefläche 6, die bei bestimmungsgemäßer Verwendung das Implantat sichert.

An die Kappe 9 grenzt der Antrieb 7 an.

Mit dem Antrieb 7 kann die Schraube 1 in ein Handhabungswerkzeug (nicht dargestellt) eingesetzt werden. Der Antrieb 7 wird dabei vorzugsweise von dem Handhabungswerkzeug so gesichert, dass er nicht herausfallen kann.

Zwischen der Oberseite 9 der Kappe 5 und dem Antrieb 7 befindet sich eine Einschnürung, welche eine Sollbruchstelle 8 bildet.

Im Bereich der Sollbruchstelle 8 ist der Durchmesser der Schraube 1 am kleinsten.

Insbesondere kann die Sollbruchstelle 8 einen 5 bis 10 % kleineren Durchmesser als die dünnste Stelle des Schaftes 4 oder des Kerndurchmessers des Gewindes 3 aufweisen.

Die Sollbruchstelle 8 befindet sich direkt angrenzend zur Kappe 5, so dass beim Abbrechen des Antriebs 7 nur noch die dann flache Kappe 5 verbleibt.

Von der Sollbruchstelle 8, welche insbesondere als Radius ausgebildet sein kann, vergrößert sich in Richtung Antrieb 7 der Durchmesser der Schraube 1, in diesem Ausführungsbeispiel in Form eines Kegelstumpfes in dem Bereich 21 mit einem Spitzenwinkel von beispielsweise 40 bis 80°.

Das Gewinde 3 ist einzügig ausgebildet. Der Gewindezug 16 läuft vorzugsweise weniger als 10, besonders bevorzugt weniger als 6 Mal um den Kern. So kann die Schraube 1 mit weinigen Umdrehungen eingedreht werden.

Die Spitze 17 der Schraube 1 ist zum einen selbstschneidend ausgebildet und mit der Schneide 14 versehen. Über die Schneide 14 werden beim Eindrehen der Schraube 1 in ein vorgebohrtes Bohrloch die Gewindezüge in den Knochen eingebracht.

Weiter hat die Spitze 17 einen Zentrierkegel 13, welcher in diesem Ausführungsbeispiel eine Mantellinie hat, die einen steileren Winkel zur Mittelachse 23 der Schraube 1 einnimmt als die Schneidkante 22 der Schneide 14.

Die Schneidkante 22 kann insbesondere einen Winkel zur Mittelachse 23 von 10 bis 30° einnehmen.

Das Gewinde 3 erstreckt sich bei diesem Ausführungsbeispiel nahezu bis zum Kopf 2 der Schraube 1. Der konusförmige Abschnitt 10 des Schaftes 4 grenzt bei diesem Ausführungsbeispiel einer Schraube 1 direkt an das Gewinde 3 an.

Angrenzend zur Unterseite, also der Anlagefläche 6 der Kappe 5, vergrößert sich der Durchmesser des Schaftes 4 vom Gewinde 3 ausgehend in Richtung der Kappe 5.

Es ist in diesem Ausführungsbeispiel ein konusförmiger Abschnitt 10 vorgesehen, welcher vorzugsweise zur Mittelachse 23 eine Mantellinie mit einem Winkel von 20 bis 40° hat.

Im Bereich des konusförmigen Abschnitts 10 vergrößert sich der Durchmesser des Schaftes 4 vorzugsweise auf das 1,2 bis 1,7fache des kleinsten Durchmessers des Schaftes 4 und/oder des Kerndurchmessers des Gewindes 3.

Der Übergangsbereich 11 vom konusförmigen Abschnitt 10 des Schaftes zum Kopf 2 hin ist in diesem Ausführungsbeispiel als Radius ausgebildet.

In dem Übergangsbereich 11, welcher insbesondere einen Radius von 0,1 bis 0,5 mm haben kann, vergrößert sich der Durchmesser sprunghaft, so dass die Schraube 1 beim Eindrehen spätestens durch diesen Übergangsbereich 11 gestoppt wird.

Der gewindeseitige Übergangsbereich 12 des konusförmigen Abschnitts 10 ist vorzugsweise abgerundet ausgebildet.

Es wird so insbesondere bei der Befestigung von biegsamen Flächengebilden wie Kollagenmembranen oder Folien verhindert, dass die Anlagefläche 6 derart auf das Implantat gepresst wird, dass es zu einer Ausstanzung kommt.

Der Durchmesser der Kappe 5 beträgt vorzugsweise 2 bis 4 mm, der Durchmesser des Schaftes beträgt vorzugsweise 0,6 bis 1,5 mm.

Der Zentrierkegel 13 kann einen Spitzenwinkel von 70 bis 110° aufweisen.

Das Gewinde der erfindungsgemäßen Knochenschraube hat vorzugsweise eine Länge von 2 bis 18 mm, bei dieser Ausführungsform einer kurzen Schraube vorzugsweise von 2 bis 5 mm.

Vorzugsweise läuft der Gewindezug 16 weniger als fünf, besonders bevorzugt weniger als vier Mal um die Schraube 1. So kann die Schraube mit nur wenigen Umdrehungen eingedreht werden.

Fig. 2 zeigt eine Draufsicht auf die Spitze der Schraube 1.

Fig. 3 ist eine Detailansicht eines Schnittes entlang der Linie A-A der Fig. 2.

Hier ist zu erkennen, dass die Gewindezüge 16 als abgerundete Einsenkungen ausgebildet sind, deren Flanken 18 vorzugsweise in einen Flankenwinkel α von 90 bis 150° einnehmen. Die Gewindezähne 15 sind vorzugsweise flach ausgebildet, insbesondere haben diese eine axiale Länge von 0,2 bis 0,5 mm.

Das Gewinde 3 ist in diesem Ausführungsbeispiel als einzügiges Trapezgewinde mit abgerundetem Zahngrund ausgebildet.

Die Breite b1 eines Gewindezahns 15 kann mindestens das 0,5fach der Breite b2 des Zahngrundes (einschließlich Zahnflanken) betragen.

Die Gewindezähne 15 bilden keine Spitze, da eine Spitze initial einer hohen Korrosion ausgesetzt wäre und so die Festigkeit der Schaubverbindung zu schnell nachlassen würde.

Fig. 4 ist eine Schnittansicht entlang der Linie B-B der Fig. 1.

Der Antrieb 7 ist bei diesem Ausführungsbeispiel im Querschnitt dreieckig mit abgerundeten Spitzen ausgebildet. Es versteht sich aber, dass die Geometrie des Antriebs 7 recht beliebig ist. Diese muss nur die Übertragung eines ausreichenden Drehmoments sicherstellen. So ist beispielsweise auch eine sechskant- oder eine sternförmige Ausgestaltung denkbar (nicht dargestellt).

Fig. 5 ist eine Seitenansicht einer alternativen Ausführungsform einer Schraube, welche länger ist als die in Fig. 1 bis Fig. 4 dargestellte Schraube. Diese hat eine Gesamtlänge von vorzugsweise mehr als 8, besonders bevorzugt mehr als 12 mm.

Die Schraube 1 umfasst auch in diesem Ausführungsbeispiel einen Kopf 2, welcher den Antrieb 7 und die flache Kappe 5 umfasst.

Der nicht mit einem Gewinde versehene Schaft 4 der Schraube 1 ist in diesem Ausführungsbeispiel mindestens doppelt, vorzugsweise mindestens dreimal so lang wie das Gewinde 3. Schaft 4 und Gewinde 3 sind vorzugsweise zusammen zwischen 8 und 18 mm lang.

Ansonsten ist die Schraube ähnlich der bezugnehmend auf Fig. 1 bis Fig. 4 dargestellten Schraube ausgebildet und umfasst insbesondere auch den konusförmigen Abschnitt 10 am kopfseitigen Ende des Schaftes 4 sowie die Spitze 17 mit einer Schneide 14.

Fig. 6 ist eine Detailansicht der Fig. 5, in welcher zu erkennen ist, dass die hier dargestellte lange Version einer Schraube 1 von der Ausgestaltung im Wesentlichen der kürzeren Version entsprechend der Zeichnungen Fig. 1 bis Fig. 4 entspricht.

Der Schaft 4 der Schraube 1 sowie der Kerndurchmesser des Gewindes 3 ist aber vorzugsweise etwas dicker. Insbesondere hat dieser einen Durchmesser von 1,0 bis 1,3 mm.

Es hat sich herausgestellt, dass bereits eine Einschnürung mit nur geringfügig kleinerem Durchmesser als der Schaft 4, insbesondere mit 0,01 bis 0,05 mm kleinerem Durchmesser, zu einer hinreichend sicheren Sollbruchstelle 8 führt.

Auch die Spitze 17 ist entsprechend der Fig. 1 ausgebildet und umfasst eine Schneide 14 zum Einbringen der Gewindezüge sowie einen Zentrierkegel 13 ohne Schneide.

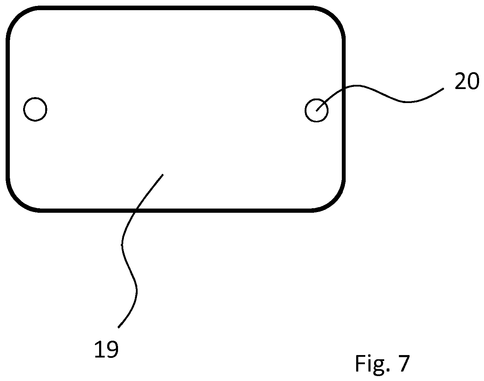

Fig. 7 ist eine schematische Ansicht eines als Flächengebilde, beispielsweise als Kollagenfolie ausgebildeten Implantats 19. Dieses wird zum Abdecken beispielsweise eines Knochendefekts auf die Wunde gelegt. Sodann werden mittels eines Bohrers Bohrlöcher 20 eingebracht, welche im Wesentlichen dem Durchmesser des Schaftes der Schraube und/oder dem Durchmesser des Gewindekerns entsprechen.

Anschließend wird das Implantat 19 mit zumindest einer Schraube befestigt.

Dabei wird die Schraube in das Bohrloch 20 hineingedreht, bis am Ende des konusförmigen Abschnitts (10 in Fig. 1 und Fig. 6) die Drehung gestoppt wird.

Der Antrieb bricht sodann entweder durch das Drehmoment beim Eindrehen ab, wenn der Kopf der Schraube mit dem Implantat bzw. dem Knochen in Kontakt kommt. Der Benutzer kann den Antrieb aber auch durch Abknicken abbrechen.

Die Schrauben bestehen vorzugsweise aus einer Magnesiumlegierung. Im Falle der Verwendung einer Magnesiumfolie als Implantat 19 kommt es aufgrund der allenfalls geringen Spannungsdifferenz nicht zu einer erhöhten Korrosion, so dass sowohl Implantat 19 als auch Schraube erst nach Heilung degradieren.

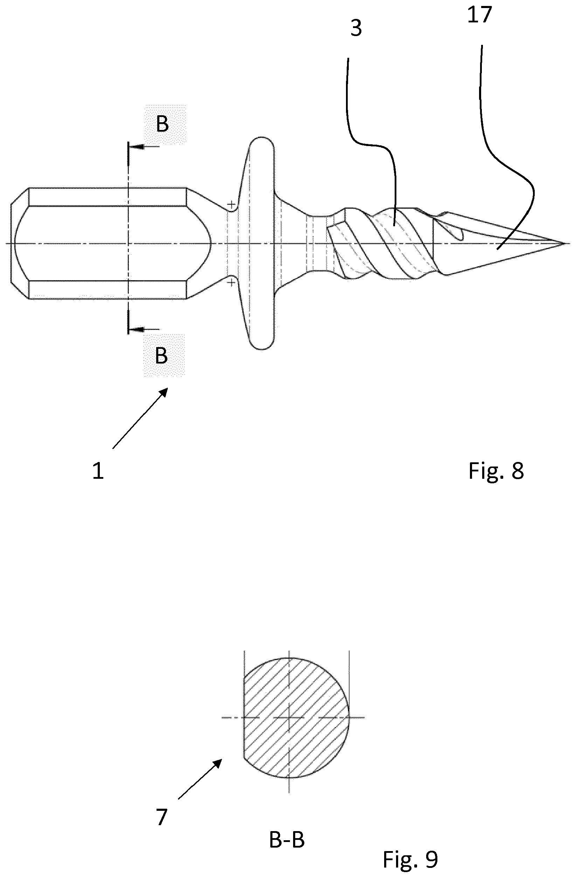

Fig. 8 und Fig. 9 zeigt eine alternative Ausführungsform einer Schraube 1, welche auch eingeschlagen werden kann.

Wie in Fig. 8 dargestellt, verläuft die selbstschneidende Spitze 17 bis zum vorderen Ende der Schraube 1. Die selbstschneidende Spitze 17 hat einen Kegelwinkel von unter 45°, vorzugsweise von unter 40°. Ein separater Zentrierkegel mit einem anderen Kegelwinkel (13 in Fig. 1) ist nicht vorgesehen.

Weiter ist das Gewinde 8 mehrzügig, insbesondere zweizügig, ausgebildet und hat eine höhere Steigung als das Gewinde der Ausführungsformen gemäß Fig. 1 bis Fig. 6.

Fig. 9 ist eine Schnittansicht entlang der Linie B-B aus Fig. 8. Der Antrieb 7 hat eine andere Geometrie als der Antrieb gemäß Fig. 1 bis Fig. 6, nämlich die eines auf einer Seite angeschnittenen Kreiszylinders. Es versteht sich aber, dass der Antrieb ebenso die die Fig. 4 dargestellte Geometrie aufweisen kann.

Ansonsten entspricht die in Fig. 8 und Fig. 9 dargestellte Schraube im Wesentlichen dem Ausführungsbeispiel gemäß Fig. 1.

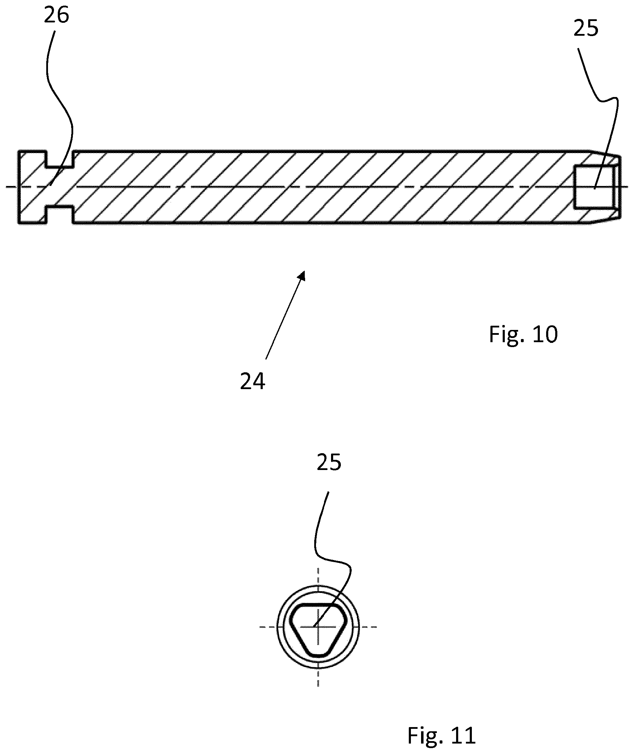

Fig. 10 ist eine Schnittansicht eines Zwischenstücks 24 für eine Schraube, welcher auf einen Dentalbohrer oder Handschraubendreher (nicht dargestellt) aufgesetzt werden kann.

Das Zwischenstück 24 umfasst hierzu eine mit dem Dentalbohrer oder Handschraubendreher verbindbare Kupplung 26, die, abgestimmt auf den verwendeten Bohrer oder Schraubendreher, beliebig ausgebildet sein kann.

Weiter umfasst das Zwischenstück, insbesondere ähnlich wie ein Bit-Halter, eine auf Aufnahme 25 für den Antrieb einer Schraube.

Wie in der Draufsicht auf die Stirnseite gemäß Fig. 11 zu erkennen ist, ist die Geometrie der Aufnahme 25 in diesem Ausführungsbeispiel auf die in Fig. 4 dargestellte Geometrie des Antriebs der Schraube abgestimmt.

Vorzugsweise kann der Antrieb der Schraube in der Aufnahme 25 verklemmt oder verrastet werden. Nach vollständigem Eindrehen der Schraube verbleibt der abgebrochene Antrieb in der Aufnahme 25.

Gemäß einer Ausführungsform (nicht dargestellt) kann der Antrieb sodann über ein Handhabungsorgan ausgeworfen werden, z.B. über einen in dem Zwischenstück 24 axial verschiebbaren Stift, der den Antrieb herausdrückt oder durch Freigabe einer Verrastung.

Durch die Erfindung wurde eine einfache und zuverlässige Befestigung, insbesondere von Dentalimplantaten, bereitgestellt.

### Bezugszeichenliste

- 1: Schraube
- 2: Kopf
- 3: Gewinde
- 4: Schaft
- 5: Kappe
- 6: Anlagefläche
- 7: Antrieb
- 8: Sollbruchstelle
- 9: Oberseite
- 10: konusförmiger Abschnitt
- 11: Übergangsbereich
- 12: Übergangsbereich
- 13: Zentrierkegel
- 14: Schneide
- 15: Gewindezahn
- 16: Gewindezug
- 17: Spitze
- 18: Flanke
- 19: Implantat
- 20: Bohrung
- 21: Bereich
- 22: Schneidkante
- 23: Mittelachse
- 24: Zwischenstück
- 25: Aufnahme
- 26: Kupplung

## Patentansprüche

1. Schraube (1) zur Fixierung eines Implantats (19), wobei die Schraube (1) aus
einem bioresorbierbaren Material, nämlich aus Magnesium oder aus einer Magnesiumlegierung besteht, wobei die Schraube (1) ein Gewinde (3) zum Eindrehen in den Knochen aufweist, wobei die Schraube (1) einen Kopf (2) mit einer Kappe (5) und einem Antrieb (7) umfasst, wobei die Kappe (5) eine im wesentlichen flache Anlagefläche für das Implantat (19) aufweist, wobei zwischen dem Antrieb (7) und der Kappe (5) eine Sollbruchstelle (8) vorhanden ist, welche als Einschnürung zwischen einer Oberseite der Kappe (5) und dem Antrieb (7) ausgebildet ist, wobei sich ein Schaft (4) der Schraube (1) zur Kappe (5) hin verdickt, wobei das Gewinde (3) derart ausgebildet ist, dass die Zähne des Gewindes (3) außen flach oder abgerundet ausgebildet sind und der Zahngrund des Gewindes (3) abgerundet in die Zahnflanken (18) übergeht, und wobei die Schraube (1) eine selbstschneidende Spitze (17) hat.

2. Schraube (1) nach dem vorstehenden Anspruch, **dadurch gekennzeichnet, dass** die Sollbruchstelle (8) als Einschnürung ausgebildet ist.

3. Schraube (1)nach einem der vorstehenden Ansprüche, **dadurch gekennzeichnet, dass** die Kappe (5) plattenförmig ausgebildet ist, vorzugsweise dass die Kappe (5) eine maximale Höhe bis zur Sollbruchstelle (8) von weniger als 2 mm, bevorzugt von weniger als 1 mm und besonders bevorzugt von weniger als 0,8 mm aufweist.

4. Schraube (1) nach einem der vorstehenden Ansprüche, **dadurch gekennzeichnet, dass** der kleinste Durchmesser des Schaftes (4) maximal so groß ist wie der Kerndurchmesser des Gewindes (3), insbesondere dass der kleinste Durchmesser des Schaftes (4) dem Kerndurchmesser des Gewindes (3) entspricht.

5. Schraube (1) nach einem der vorstehenden Ansprüche, **dadurch gekennzeichnet, dass** die Kappe (5) einen Durchmesser von 1 bis 6 mm, vorzugsweise von 2 bis 4 mm hat
und/oder dass die Kappe (5) den 1,5 bis 5fachen, vorzugsweise den 2 bis 4fachen Durchmesser des Schaftes (4)
und/oder Kerns des Gewindes (3) hat
und/oder dass die Steigung des Gewindes (3) 0,5 bis 1,6 mm beträgt
und/oder dass die Schraube (1) eine Zentrierspitze aufweist.

6. Schraube (1)nach einem der vorstehenden Ansprüche, **dadurch gekennzeichnet, dass** die Schraube (1) aus einer Magnesiumlegierung besteht, insbesondere einer Magnesiumlegierung mit Yttrium, Zink, Mangan und/oder Calcium.

7. Schraube (1) nach einem der vorstehenden Ansprüche, **dadurch gekennzeichnet, dass** die Schraube (1) eine Beschichtung aufweist, insbesondere eine Beschichtung aus Magnesiumfluorid.

8. Schraube (1) nach einem der vorstehenden Ansprüche, **dadurch gekennzeichnet, dass** der Schaft (4)angrenzend zum Kopf (2) einen konusförmigen, insbesondere kegelstumpfförmigen, Abschnitt aufweist.

9. Schraube (1)nach dem vorstehenden Anspruch, **dadurch gekennzeichnet, dass** der Winkel des konusförmigen Abschnitt in einem Übergangsbereich zur Kappe (5)hin bezogen auf eine Mittelachse steiler wird, insbesondere dass der Übergangsbereich (11) als Radius ausgebildet ist.

10. Schraube (1) nach dem vorstehenden Anspruch, **dadurch gekennzeichnet, dass** der Übergangsbereich (11) eine Länge von 0,5 bis 2 mm hat.

11. Schraube (1) nach einem der vorstehenden Ansprüche, **dadurch gekennzeichnet, dass** die Schraube (1) unterhalb der Kappe (5)einen konusförmigen Abschnitt aufweist, welcher einen Winkel bezogen auf die Mittelachse der Schraube (1) von 20 bis 40°, vorzugsweise von 35 bis 45° einnimmt und/oder welcher eine Länge von 0,2 bis 10 mm, vorzugsweise von 0,4 bis 0,6 mm, hat.

12. Set mit zumindest einer Schraube (1) nach einem der vorstehenden Ansprüche und einem Implantat (19), welches als Flächengebilde ausgebildet ist.

13. Set nach dem vorstehenden Anspruch, weiter umfassend einen Vorbohrer, welcher vorzugsweise in etwa den Durchmesser des Schafts (4) der Schraube (1) aufweist, und/oder ein Zwischenstück zur Aufnahme einer Schraube (1), wobei das Zwischenstück eine Kupplung (26)zur Verbindung mit einem Handstück oder mit einem Dentalbohrer aufweist, und/oder einen Halter für eine Mehrzahl von Schrauben.

## Claims

1. A screw (1) for fixing an implant (19), whereby the screw consists of a bioresorbable material, namely of magnesium or of a magnesium alloy,
whereby the screw (1) comprises a thread (3) for screwing it into the bone, whereby the screw (1) comprises a head (2) with a cap (5) and a drive (7), whereby the cap (5) comprises an essentially flat contact surface for the implant (19), whereby a predetermined breaking point (8) that is designed as a constriction between a top side of the cap (5) and the drive (7) is present between the drive (7) and the cap (5), and whereby a shaft (4) of the screw thickens towards the cap (5), and whereby the thread (3) is embodied in that the teeth of the thread (3) are designed to be flat or rounded on the outside and wherein the tooth base of the thread (3) merges rounded into the tooth flanks (18) and wherein the thread (3) has a self-tapping tip (17).

2. The screw (1) according to the preceding claims, **characterised in that** the predetermined breaking (8) point is designed as a constriction.

3. The screw (1) according to any one of the preceding claims, **characterised in that** the cap (5) is provided to be plate-shaped, preferably **in that** the cap (5) comprises a maximum height up to the predetermined breaking point (8) of less than 2 mm, more preferably of less than 1 mm and particularly preferably of less than 0.8 mm.

4. The screw (4) according to any one of the preceding claims, **characterised in that** the smallest diameter of the shaft (4) is at most as large as the core diameter of the thread (3), in particular **in that** the smallest diameter of the shaft (4) is equivalent to the core diameter (3) of the thread.

5. The screw (1) according to any one of the preceding claims, **characterised in that** the cap (3) has a diameter of 1 to 6 mm, preferably of 2 to 4 mm
and/or **in that** the diameter of the cap (5) is 1.5 to 5 times, preferably 2 to 4 times the diameter of the shaft (4)
and/or core of the thread (3)
and/or **in that** the pitch of the thread (3) is 0.5 to 1.6 mm
and/or **in that** the screw (1) comprises a centring tip.

6. The screw (1) according to any one of the preceding claims, **characterised in that** the screw consists of a magnesium alloy, in particular a magnesium alloy containing yttrium, zinc, manganese and/or calcium.

7. The screw (1) according to any one of the preceding claims, **characterised in that** the screw (1) comprises a coating, in particular a coating made of magnesium fluoride.

8. The screw (1) according to any one of the preceding claims, **characterised in that** the shaft (4) comprises a cone-shaped, in particular truncated cone-shaped, section adjacent to the head (2).

9. The screw (1) according to the preceding claim, **characterised in that** the angle of the cone-shaped section becomes steeper in a transition region towards the cap (5), with respect to a central axis, in particular **in that** the transition region (11) is designed as a radius.

10. The screw (1) according to the preceding claim, **characterised in that** the transition region (11) has a length of 0.5 to 2 mm.

11. The screw (1) according to any one of the preceding claims, **characterised in that** the screw (1) comprises, below the cap (5), a cone-shaped section which is at an angle of 20 to 40°, preferably of 35 to 45°, with respect to the central axis of the screw (1), and/or which has a length of 0.2 to 10 mm, preferably of 0.4 to 0.6 mm.

12. A kit including at least one screw (1) according to any one of the preceding claims and an implant (19) that is designed as a sheet-like structure.

13. The kit according to the preceding claim, further comprising a pilot drill, which preferably has approximately the same diameter as the shaft (4) of the screw (1), and/or an adapter for holding a screw (1), whereby the adapter comprises a coupling (26) for connection to a handpiece or to a dental drill, and/or a holder for a plurality of screws.

## Revendications

1. Vis (1) pour la fixation d'un implant (19), la vis (1) étant constituée d'un matériau biorésorbable, à savoir de magnésium ou d'un alliage de magnésium, la vis (1) présentant un filetage (3) à visser dans l'os, la vis (1) comprenant une tête (2) avec un capuchon (5) et un entraînement (7), le capuchon (5) présentant une surface d'appui essentiellement plate pour l'implant (19), un point de rupture (8) étant présent entre l'entraînement (7) et le capuchon (5), lequel est réalisé sous forme de rétrécissement entre une face supérieure du capuchon (5) et l'entraînement (7), une tige (4) de la vis (1) s'épaississant en direction du capuchon (5), le filetage (3) étant réalisé de manière à ce que les dents du filetage (3) soient plates ou arrondies à l'extérieur et que le fond de dent du filetage (3) s'arrondisse dans les flancs des dents (18), et la vis (1) présentant une pointe autotaraudeuse (17).

2. Vis (1) selon la revendication précédente, **caractérisée en ce que** le point de rupture (8) est réalisé sous forme de rétrécissement.

3. Vis (1) selon l'une des revendications précédentes, **caractérisée en ce que** le capuchon (5) est réalisé en forme de plaque, de préférence **en ce que** le capuchon (5) présente une hauteur maximale jusqu'au point de rupture (8) de moins de 2 mm, préférablement de moins de 1 mm et plus préférablement de moins de 0,8 mm.

4. Vis (1) selon l'une des revendications précédentes, **caractérisée en ce que** le plus petit diamètre de la tige (4) est au maximum aussi grand que le diamètre du noyau du filetage (3), en particulier **en ce que** le plus petit diamètre de la tige (4) correspond au diamètre du noyau du filetage (3).

5. Vis (1) selon l'une des revendications précédentes, **caractérisée en ce que** le capuchon (5) a un diamètre de 1 à 6 mm, de préférence de 2 à 4 mm,
et/ou que le capuchon (5) a un diamètre de 1,5 à 5 fois, de préférence de 2 à 4 fois, celui de la tige (4)
et/ou du noyau du filetage (3),
et/ou **en ce que** le pas du filetage (3) est de 0,5 à 1,6 mm,
et/ou **en ce que** la vis (1) présente une pointe de centrage.

6. Vis (1) selon l'une des revendications précédentes, **caractérisée en ce que** la vis (1) est constituée d'un alliage de magnésium, en particulier d'un alliage de magnésium avec de l'yttrium, du zinc, du manganèse et/ou du calcium.

7. Vis (1) selon l'une des revendications précédentes, **caractérisée en ce que** la vis (1) présente un revêtement, en particulier un revêtement en fluorure de magnésium.

8. Vis (1) selon l'une des revendications précédentes, **caractérisée en ce que** la tige (4) présente une section conique, en particulier tronconique, adjacente à la tête (2).

9. Vis (1) selon la revendication précédente, **caractérisée en ce que** l'angle de la section conique dans une zone de transition vers le capuchon (5) par rapport à un axe central est plus prononcé, en particulier **en ce que** la zone de transition (11) est conçue comme un rayon.

10. Vis (1) selon la revendication précédente, **caractérisée en ce que** la zone de transition (11) a une longueur de 0,5 à 2 mm.

11. Vis (1) selon l'une des revendications précédentes, **caractérisée en ce que** la vis (1) présente, sous le capuchon (5), une section conique qui forme un angle de 20 à 40°, de préférence de 35 à 45°, par rapport à l'axe central de la vis (1) et/ou qui a une longueur de 0,2 à 10 mm, de préférence de 0,4 à 0,6 mm.

12. Ensemble comprenant au moins une vis (1) selon l'une des revendications précédentes et un implant (19) qui est conçu comme une structure plane.

13. Ensemble selon la revendication précédente, comprenant en outre un foret d'amorçage qui présente de préférence à peu près le diamètre de la tige (4) de la vis (1), et/ou une pièce intermédiaire destinée à recevoir une vis (1), la pièce intermédiaire présentant un raccord (26) pour être couplée à une pièce à main ou à un foret dentaire, et/ou un support pour une pluralité de vis.
